# EUROPEAN PATENT SPECIFICATION

(11) **EP 1 371 325 B1**
(45) Date of publication and mention of the grant of the patent: **12.01.2005**
(21) Application number: 02405490.0
(22) Date of filing: 14.06.2002
(51) Int. Cl.: A61B 5/0215, G01L 9/18, A61B 5/06

(54) **Sensor for measuring a pressure profile**
Sensor zur Messung eines Druckprofils
Détecteur pour mesurer un profil de pression

(43) Date of publication of application: 17.12.2003
(73) Proprietor: PP-Technologies AG, 3186 Düdingen (CH)
(72) Inventor: Glocker, Raymond, 3186 Düdingen (CH); Özdemir, Haldun, 3186 Düdingen (CH)
(74) Representative: Besse, François

(56) References cited:
- EP-A- 0 990 883
- WO-A-84/00290
- DE-C- 19 724 001

## Description

The present invention relates to a pressure sensing device and to a method for using said device.
More precisely the invention concerns a device for measuring pressure profiles in mammals.

The Pressure Profile Sensor Method, defined as PPS-Method hereafter is disclosed in German patent DE 197 24 001 C1. It is based on the deformation applied by external pressure to a catheter which contains a saline solution. As the saline solution is electrically conducting, the impedance of the saline solution changes when the shape is changed through the applied pressure along the axis of the tubing (co-ordinates x) and over time (co-ordinates t). The most sensitive part of the saline solution column - to change the shape - is the front of the saline solution. That means the pressure profile (x) can be measured over time (therefore the word pressure profile sensor), p over x and t, p(x,t).

The inert mass of the saline solution column - being by total volume incompressible as all standard fluids - allows expansion only at the front of the saline solution column. At this end only a little inert mass needs to be moved by the force generated by the external pressure. The column portions being far away from the front need to move all the other column portions towards that front which results into continuously higher inert masses to be moved. The external pressure is not capable to do that after a certain very limited extend. To avoid a gas spring effect, the front of the saline solution can move forward in the saline solution lumen (with thin wall) connected to a venting lumen, that means at the tip of the PPS-catheter these two lumens are connected to each other. All that measures result into the situation that the front of the saline solution column has to be the place of measurement. The external pressure is causing there the geometrical changes of the shape of the saline solution resulting into a change of the impedance of the full saline solution column which can be measured by the PPS-machine and translated through an algorithm described in patent DE 197 24 001 C1 and in publication "Pressure Profile Sensing system" in Sensors and Actuators A93 (2001), 52-56, Elsevier Science B.V.

To find the pressure signal with the pressure sensitive front of the saline solution, the machine to control the placement of the saline solution column and to measure the impedance is equipped with hardware as well as software to correlate the pressure curves p(t) with the various locations x, a so-called pressure signal finding mode, abbr. PSFM.

If the tubing is a catheter inserted into an organism through e.g. a vessel then the user cannot see where the front of the saline solution column is located. This situation makes it very difficult and often impossible to correlate the pressure signals with the location x of the axis of the catheter.

The fast variations of pressure in the circulatory system are caused by the heart beating usually a little bit faster than one beat per second. The pressure profile over t can typically be displayed correctly if the measurement has a natural frequency response in the range of 20 Hz. That is usually also the response frequency of conventional pressure monitoring systems where the pressure signal enters into the tip opening of a catheter travelling through the inner lumen of the catheter into the saline solution filled tubing of the pressure monitoring set till the electrical chip-based transducer outside the body. And that has been proven as sufficient for already several decades. Only to mention the just described system of pressure recording can only measure the time profile of the pressure at the tip of the catheter. Only the pressure at one single place, not the pressure at the various locations along the length of the catheter or even only a small portion of it.

As already mention, when the PPS machine (with pump) is pushing the saline solution column into the catheter, the position of the front cannot be located when the catheter is "in-situ". The filling situation could be a measure if all the tubing (mainly from the transfer set with much higher filling volume) was exactly known with respect to its inner diameter. But this approach is unrealistic since the normal tolerances for such tubing of the transfer set are relatively high.

One of the major objectives of the present invention aims at measuring p(t) at different locations in an organism, i.e. where the front of the saline solution cannot be seen.

For that purpose the invention makes use of the advantage that in the organism the pressure curves p(t) can be correlated with the potential different locations of the catheter tips during insertion of the catheter, see e.g. "Essentials in Pressure Monitoring" by J. Cywinski, ISBN902472385X, pages 92 and 93, figures 18 and 19 (see drawings).

The invention therefore relates to a pressure sensing device as defined in claim 1.

The basic concept of the invention is characterized by the fact the conductive solution column oscillates, preferably at about 20 Hz around search points x₀, resp. x₁ etc. to measure the pressure at that points. The oscillation can be generated by a special configuration of the motor and the pump head as well as the collection of the data p(x₀), resp. p(x₁) at running times t and then the display of p(t) at location x₀, resp. x₁ in real-time based on a special configuration of the algorithm of the program of the PPS method. It is important to keep the oscillating distance short (few mm) as otherwise the fast oscillation will cut or destroy the fluid column.

This procedure shall be applied step by step at proceeding locations x₀, x₁, x₂, (moving the front of the saline solution column towards the distal tip of the catheter). The pressure curves p(t) can then be displayed step by step (not simultaneously) at the locations x₀, x₁, x₂. As the physician knows the typical pressure curves from the conventional kind of pressure monitoring , and there also with a resolution of 20 Hz, he can judge the location of the front of the saline solution column from the shape of the displayed pressure curve. This works independent from the potential calculations about the filling status of the PPS-transfer-set and the PPS-catheter and is more reliable than that. And exactly this correlation of the displayed p(t)-curves to the location of the front of the saline solution column is PSFM, the pressure signal finding mode.

When the catheter is filled according to the intended position, this position will be defined as "zero" and from there slow oscillations can scan the catheter in a wide range (several cm) in case the oscillation frequency is slow enough (e.g. 1/10 Hz) not to disturb the saline solution homogeneity due to the oscillations.

The recording of the impedance signal of the oscillating saline solution column according to the PPS method will be realised in real time that an analogue differentiator circuit is deriving from the leakage current the pressure value at the oscillating place x immediately, displaying these values on the ordinates continuing on the horizontal axis and therefore writing the curve p(t) at place x. This special measurement mode then allows the user to compare such p(t) curves with physiological wave forms to correlate to the measurement location x in terms of anatomy.

The pump head of the peristaltic pump can move a very small volume of 1-2 mm³ by using a pump segment tubing of typically 1-1.5 mm inner diameter - much smaller than typically used for such pump segments for feeding infusion solutions for infusion therapy - to allow fast oscillations of the saline solution column up to 25 Hz.

This further comment is not related to PSFM, only to explain the kind of measurement following after PSFM:

To display the pressure curves p(t) at different locations "simultaneously" the oscillation shall have a small defined phase shift to the EKG and therefore to the pressure waves coming from the heart. The data will be collected till a full cycle of phase shifts will add up to a full period and then re-addressing the data p(xᵢ) at ongoing times tj to p(t, frequency resolution 20 Hz) at different locations xᵢ. But as described, that is not fully real-time, the collection of data and re-addressing over e.g. 20 pressure cycles is necessary (for the frequency resolution of 20 Hz), that means around 20 seconds of measurement time.

For the (nearly) real time display of p(t) we cannot use this method in the paragraph above, the fast oscillating method must be used.. In patent DE 197 24 001 C1 a syringe pump is described driven by an excenter and a push-pull mechanic. Our own experiments which such kind of a construction show that even with best versions of syringes only a maximum oscillation frequency of 10 Hz can be achieved.

In addition the filling situation of the syringe and the connection tubing without air bubbles is very difficult to achieve. The normal user knows the procedure to fill standard pressure monitoring sets only from straight-through configurations, and even that is difficult. He would like to find such a situation again in the PPS method

It is therefore a preferred embodiment of this invention, based on the principles of the patent DE 197 24 001 C1 to use a peristaltic pump instead of a syringe pump plus replacing the linear motor - used normally to drive peristaltic pumps to get reliable feeding - by a stepper motor which can be triggered fast and discrete to fill the PPS-transfer-set plus PPS-catheter and to create fast as well as slow oscillations.

## Claims

1. Pressure sensing device for measuring pressure profiles comprising :
- a flexible tubing,
- actuating means for moving a conductive solution within said flexible tubing,
- impedance measuring means for measuring the impedance of said solution,
- processing means for transforming an impedance profile into a pressure profile,
**characterized by** the fact that it furthermore comprises :
- conductive solution oscillating means.

2. Pressure sensing device according to claim 1 wherein said actuating means comprises a stepper motor.

3. Pressure sensing device according to claim 2 wherein said actuating means furthermore comprises a peristaltic pump.

4. Pressure sensing device according to claim 3 wherein the peristaltic pump segment is directly connected to said flexible tubing in such a way that pressure can be measured.

5. Pressure sensing device according to any of the previous claims comprising display means to display the geometry of said flexible tubing.

6. Pressure sensing device according to any of the previous claims comprising filling status means for indicating the approximate position of the front of the conductive solution.

## Patentansprüche

1. Druckmessvorrichtung zur Messung von Druckprofilen, umfassend:
- einen flexiblen Schlauch,
- Betätigungsmittel zum Bewegen einer leitenden Lösung in dem flexiblen Schlauch,
- Impedanzmessmittel zur Messung der Impedanz der Lösung,
- Verarbeitungsmittel zur Umwandlung eines Impedanzprofils in ein Druckprofil,
**gekennzeichnet durch** die Tatsache, dass sie ferner
- ein Oszillationsmittel in einer leitenden Lösung umfasst.

2. Druckmessvorrichtung nach Anspruch 1, worin das Betätigungsmittel einen Schrittmotor umfasst.

3. Druckmessvorrichtung nach Anspruch 2, worin das Betätigungsmittel ferner eine Peristaltikpumpe umfasst.

4. Druckmessvorrichtung nach Anspruch 3, worin das Peristaltikpumpensegment direkt mit dem flexiblen Schlauch verbunden ist, so dass der Druck gemessen werden kann.

5. Druckmessvorrichtung nach einem der vorhergehenden Ansprüche, umfassend ein Anzeigemittel zum Anzeigen der Geometrie des flexiblen Schlauchs.

6. Druckmessvorrichtung nach einem der vorhergehenden Ansprüche, umfassend ein Füllzustandsmittel zur Anzeige der ungefähren Position der Vorderseite der leitenden Lösung.

## Revendications

1. Dispositif de détection de pression pour mesurer des profils de pression, qui comprend:
un tuyau souple,
un moyen d'actionnement pour déplacer une solution conductrice dans ledit tuyau souple,
un moyen de mesure de l'impédance pour mesurer l'impédance de ladite solution,
un moyen de traitement pour transformer un profil d'impédance en un profil de pression,
**caractérisé par le fait qu'**il comprend en outre:
un moyen de mise en oscillation de la solution conductrice.

2. Dispositif de détection de pression selon la revendication 1, dans lequel ledit moyen d'actionnement comprend un moteur pas-à-pas.

3. Dispositif de détection de pression selon la revendication 2, dans lequel ledit moyen d'actionnement comprend en outre une pompe péristaltique.

4. Dispositif de détection de pression selon la revendication 3, dans lequel le segment de pompe péristaltique est directement relié audit tuyau souple de telle sorte que la pression puisse être mesurée.

5. Dispositif de détection de pression selon l'une quelconque des revendications précédentes, qui comprend un moyen d'affichage pour afficher la géométrie dudit tuyau souple.

6. Dispositif de détection de pression selon l'une quelconque des revendications précédentes, qui comprend un moyen de détermination de l'état de remplissage pour indiquer la position approximative du front de la solution conductrice.
